# EUROPÄISCHE PATENTANMELDUNG

(11) **EP 2 602 328 A1**
(43) Veröffentlichungstag der Anmeldung: **12.06.2013**
(21) Anmeldenummer: 11191910.6
(22) Anmeldetag: 05.12.2011
(51) Int. Cl.: C12P 7/02, C12P 7/24, C12P 7/40

(54) **Verfahren zur Oxidation von Alkanen unter Verwendung einer AlkB Alkan 1-Monooxygenase**

(71) Anmelder: Evonik Industries AG, 45128 Essen (DE)
(72) Erfinder: Pfeffer, Dr Jan Christoph, 45355 Essen (DE); Haas, Dr Thomas, 48161 Münster (DE); Thum, Dr Oliver, 40880 Ratinger (DE); Erhardt, Dr Frank, 33604 Bielefeld (DE); Wittmann, Eva-Maria, 83301 Traunreut (DE)

(57) **Zusammenfassung**

Die Erfindung betrifft ein Verfahren zur Oxidation eines Alkans umfassend Kontaktieren des Alkans mit einer Oxidoreduktase vom AlkB-Typ sowie Verwendung einer Oxidoreduktase vom AlkB-Typ zur Herstellung eines Gemisches von Oxidationsprodukten eines Alkans, wobei das Verhältnis von Carbonsäure zu Alkohol unter den Oxidationsprodukten bevorzugt größer als 1:1 ist.

## Beschreibung

Die Erfindung betrifft ein Verfahren zur Oxidation eines Alkans umfassend Kontaktieren des Alkans mit einer Oxidoreduktase vom AlkB-Typ sowie Verwendung einer Oxidoreduktase vom AlkB-Typ zur Herstellung eines Gemisches von Oxidationsprodukten eines Alkans, wobei das Verhältnis von Carbonsäure zu Alkohol unter den Oxidationsprodukten bevorzugt größer als 1:1 ist.

Alkane stellen einige der wichtigsten Grundstoffe der chemischen Industrie dar. Ausgangspunkt ihrer Gewinnung sind in der Regel fossile Rohstoffe, mittlerweile sind allerdings auch Verfahren zur Gewinnung von Alkanen aus nachwachsenden Rohstoffen bekannt. Während der Öffentlichkeit Alkane insbesondere aufgrund ihrer Verwendbarkeit als Energieträger, beispielsweise in Form von Gasen der kurzkettigen Alkane oder flüssigen längerkettigen Alkane bekannt sind, sind sie in der Industrie vor allem in ihrer Rolle als Edukte oder Lösungsmittel für zahlreiche Synthesen, die zu wichtigen Produkten des täglichen Lebens wie Kunststoffen oder Pharmazeutika führen, unentbehrlich.

Eine Grundbedingung für die Verwendung von Alkanen zu derartigen Zwecken ist die Möglichkeit der oxidativen Einführung von heteroatomhaltigen Funktionen in die Alkan-Kohlenstoffketten, z.B. von Hydroxy-, Keto- und Carboxyfunktionen, da Alkane *per se* aufgrund ihrer Sättigung chemisch als relativreaktionsträge zu charakterisieren sind. Dabei darf es, anders als bei der Verwendung von Alkanen als Heizmittel, jedoch nicht zu einer vollständigen Oxidation von Alkanen zu Kohlendioxid kommen, sondern die heteroatomhaltigen Funktionen müssen selektiv und kontrolliert eingeführt werden.

Zur synthetischen Darstellung von mit heteroatomhaltigen Funktionen substituierten Alkanen sind zahlreiche Reaktionen bekannt, beispielsweise die Halogenierung von Alkanen unter Einwirkung von UV-Licht, deren Produkte als Edukte für Synthese zahlreicher Verbindungen dienen können. So können Alkohole durch nukleophile Substitution des halogensubstituierten Alkans erhalten werden. Derartige Reaktionen verlangen jedoch häufig die Verwendung von giftigen und/oder umweltschädlichen Stoffe, beispielsweise von Chlorgas, das aufgrund seines niedrigen Preises industriell häufig zur Halogenierung eingesetzt wird.

Auch eine Reihe von biotechnologischen Verfahren zur Einführung von heteroatomhaltigen, insbesondere sauerstoffhaltigen, Funktionen in Alkane sind bekannt. So wird in einem Patent von Exxon (EP 98137) die Umsetzung von Propan zu Aceton durch *Arthrobacter petroleophagus* und andere Wildtypstämme beschrieben. Grant *et al.* (2011) verwenden rekombinanten E. coli-Zellen, um langkettige Alkane zu oxidieren.

Vor diesem Hintergrund besteht die der Erfindung zugrunde liegende Aufgabe darin, ein biotechnologisches Verfahren zur Oxidation von Alkanen zu entwickeln, das zur selektiven Oxidation eines endständigen Kohlenstoffatoms eines Alkans bis hin zur Carbonsäure geeignet ist.

Weiterhin besteht die der Erfindung zugrunde liegende Aufgabe darin, ein Verfahren zu entwickeln, dass geeignet ist, um verschiedene selektiv am endständigen Kohlenstoffatom oxidierte Produkte des Alkans herzustellen, wobei die Menge bzw. das Verhältnis der Produkte beeinflusst werden kann.

Weiterhin besteht die der Erfindung zugrunde liegende Aufgabe darin, ein Oxidoreduktasesystem bereitzustellen, das die Oxidation eines endständigen Kohlenstoffatoms zu sämtlichen Oxidationsstufen aus der Gruppe umfassend Alkohol, Aldehyd und Carbonsäure zu katalysieren imstande ist, wobei nur ein einziges katalytisch aktives Polypeptid mit dem Substratalkan bzw. dessen Zwischenprodukten in Kontakt tritt.

Weiterhin besteht die der Erfindung zugrunde liegende Aufgabe darin, ein vom Fettsäurestoffwechsel unabhängiges und durch Überexpression eines einzelnen Oxidationssystems charakterisiertes System zur selektiven endständigen Oxidation von Alkanen bereitzustellen.

Eine weitere der Erfindung zu Grunde liegenden Aufgabe besteht darin, ein Verfahren zur Oxidation von Alkanen, bevorzugt von gasförmigen Alkanen, bereitzustellen, das geeignet ist, um das Alkan überwiegend bzw. mit verbesserter Ausbeute zur Carbonsäure, nicht lediglich überwiegend zum Alkohol zu oxidieren.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

In einem ersten Aspekt wird das der Erfindung zu Grunde liegende Problem gelöst durch ein Verfahren zur Oxidation eines Alkans umfassend Kontaktieren des Alkans mit einer Oxidoreduktase vom AlkB-Typ in Anwesenheit von Sauerstoff.

In einer ersten Ausführungsform des ersten Aspektes handelt es sich bei dem Alkan um ein Alkan mit 1 bis 5 Kohlenstoffatomen.

In einer zweiten Ausführungsform des ersten Aspektes, bei der es sich auch um eine Ausführungsform der ersten Ausführungsform des ersten Aspektes handelt, handelt es sich bei dem Alkan um ein Alkan mit 1 bis 4 Kohlenstoffatomen, bevorzugt um Butan, handelt.

In einer dritten Ausführungsform des ersten Aspektes, bei der es sich auch um eine Ausführungsform der ersten bis zweiten Ausführungsform des ersten Aspektes handelt, handelt es sich bei dem Alkan um ein verzweigtes Alkan, bevorzugt mit vier oder fünf Kohlenstoffatomen, noch bevorzugter Isobutan, handelt.

In einer vierten Ausführungsform des ersten Aspektes, bei der es sich um eine Ausführungsform der ersten bis dritten Ausführungsform des ersten Aspektes handelt, handelt es sich bei der Oxidoreduktase vom AlkB-Typ um AlkB aus *Pseudomonas putida* GPO1 oder eine Variante davon handelt.

In einer fünften Ausführungsform des ersten Aspektes, bei der es sich auch um eine Ausführungsform der ersten bis vierten Ausführungsform des ersten Aspektes handelt, wird die Oxidoreduktase vom AlkB-Typ in Form von einem Ganzzellkatalysator bereitgestellt.

In einer sechsten Ausführungsform des ersten Aspektes, bei der es sich auch um eine Ausführungsform der ersten bis fünften Ausführungsform des ersten Aspektes handelt, wird die Oxidoreduktase vom AlkB-Typ in Form von einem aufgereinigten Polypeptid bereitgestellt.

In einem zweiten Aspekt wird das der Erfindung zu Grunde liegende Problem gelöst durch die Verwendung einer Oxidoreduktase vom AlkB-Typ zur Herstellung in Anwesenheit von Sauerstoff eines Gemisches von Oxidationsprodukten eines Alkans, wobei das Verhältnis von Carbonsäure zu Alkohol unter den Oxidationsprodukten bevorzugt größer als 1:1 ist.

In einer ersten Ausführungsform des zweiten Aspektes handelt es sich bei dem Alkan um ein Alkan mit 1 bis 5 Kohlenstoffatomen.

In einer zweiten Ausführungsform des zweiten Aspektes, bei der es sich auch um eine Ausführungsform der ersten Ausführungsform des zweiten Aspektes handelt, handelt es sich bei dem Alkan um ein Alkan mit 1 bis 4 Kohlenstoffatomen, bevorzugt um Butan.

In einer dritten Ausführungsform des zweiten Aspektes, bei der es sich auch um eine Ausführungsform der ersten bis zweiten Ausführungsform des zweiten Aspektes handelt, handelt es sich bei dem Alkan um ein verzweigtes Alkan, bevorzugt mit vier oder fünf Kohlenstoffatomen, noch bevorzugter Isobutan.

In einer vierten Ausführungsform des zweiten Aspektes, bei der es sich auch um eine Ausführungsform der ersten bis dritten Ausführungsform des zweiten Aspektes handelt, handelt es sich bei der Oxidoreduktase vom AlkB-Typ um AlkB aus *Pseudomonas putida* GPO1 oder eine Variante davon.

In einer fünften Ausführungsform des zweiten Aspektes, bei der es sich auch um eine Ausführungsform der ersten bis vierten Ausführungsform des zweiten Aspektes handelt, wird die Oxidoreduktase vom AlkB-Typ in Form von einem Ganzzellkatalysator bereitgestellt.

In einer sechsten Ausführungsform des zweiten Aspektes, bei der es sich auch um eine Ausführungsform der ersten bis vierten Ausführungsform des zweiten Aspektes handelt, wird die Oxidoreduktase vom AlkB-Typ in Form von einem aufgereinigten Polypeptid bereitgestellt.

In einer siebten Ausführungsform des zweiten Aspektes, bei der es sich auch um eine Ausführungsform der ersten bis sechsten Ausführungsform des zweiten Aspektes handelt, ist das Verhältnis von Carbonsäure zu Alkohol unter den Oxidationsprodukten größer als 5:1, bevorzugt größer als 12:1 ist, noch bevorzugter größer als 20:1, am bevorzugtesten größer als 40:1 ist.

Die Erfinder der vorliegenden Erfindung haben festgestellt, dass Oxidoreduktasen vom AlkB-Typ, die in der Literatur als Katalysatoren zur Herstellung überwiegend von weniger stark oxidierten Produkten bekannt sind, überraschend verwendet werden können, um aus Alkanen, insbesondere aus gasförmigen Alkanen, überwiegend Produkte höherer Oxidationsstufen, insbesondere Carbonsäuren, ausgehend von Alkanen herzustellen. Insbesondere ist das Verhältnis von produzierten Carbonsäuren zu produzierten Alkoholen überraschend hoch. Weiterhin haben die Erfinder überraschend gefunden, dass derartige Oxidoreduktasen zur selektiven Oxidation von Alkanen befähigt sind, und zu erwartende Nebenprodukte, insbesondere an anderen als endständigen Kohlenstoffatomen oxidierte Alkane nur in unerwartet geringem Ausmaß oder überhaupt nicht in nachweisbaren Mengen herstellen.

Erfindungsgemäß werden Alkane, bevorzugt gasförmige Alkane unter Verwendung einer Oxidoreduktase vom AlkB-Typ in Anwesenheit von Sauerstoff oxidiert. AlkB stellt eine zunächst aus dem AlkBGT-System aus *Pseudomonas putida* Gpo1 bekannt gewordene Oxidoreduktase dar, die von zwei weiteren Polypeptiden, AlkG und AlkT, abhängig ist. AlkT wird als FADabhängige Rubredoxin-Reduktase charakterisiert, die Elektronen aus NADH an AlkG weitergibt. Bei AlkG handelt es sich um ein Rubredoxin, ein eisenhaltiges Redoxprotein, das als direkter Elektronendonor für AlkB fungiert. In einer bevorzugten Ausführungsform wird unter dem selben Begriff "Oxidoreduktase des alkB-Typs" ein Polypeptid mit einer Sequenzhomologie von zunehmend bevorzugt wenigstens 75, 80, 85, 90, 92, 94, 96, 98 oder 99 % zur Sequenz des AlkB von *Pseudomonas putida* Gpo1 (Datenbankcode: CAB54050.1; dieser Datenbankcode stammt wie alle weiteren in der Anmeldung verwendeten aus dem Stand der Technik, nämlich aus der NCBI Datenbank, genauer dem am 15. November 2011 online verfügbaren Release) mit der Fähigkeit, Alkane zu oxidieren. In einer besonders bevorzugten Ausführungsform handelt es sich bei der Oxidoreduktase vom AlkB-Typ um eine mit den AlkG (CAB54052.1)- und AlkT (CAB54063.1)-Polypeptiden aus *Pseudomonas putida* Gpo1 funktionell zusammenwirkende, Alkane oxidierende Oxidoreduktase. In einer bevorzugtesten Ausführungsform handelt es sich bei der Oxidoreduktase vom AlkB-Typ um AlkB aus dem AlkBGT-System aus *Pseudomonas putida* Gpo1 oder eine Variante davon.

Die Lehre der vorliegenden Erfindung kann nicht nur unter Verwendung der exakten Aminosäure- oder Nukleinsäuresequenzen der hierin beschriebenen biologischen Makromoleküle ausgeführt werden, sondern auch unter Verwendung von Varianten derartiger Makromoleküle, die durch Deletion, Addition oder Substitution einer oder mehr als einer Aminosäuren oder Nukleinsäuren erhalten werden können. In einer bevorzugten Ausführungsform bedeutet der Begriff "Variante" einer Nukleinsäuresequenz oder Aminosäuresequenz, im Folgenden gleichbedeutend und austauschbar mit dem Begriff "Homologen" gebraucht, wie hierin verwendet, eine andere Nukleinsäure- oder Aminosäuresequenz, die mit Hinblick auf die entsprechende ursprüngliche Wildtyp-Nukleinsäure- oder -aminosäuresequenz eine Homologie, hier gleichbedeutend mit Identität verwendet, von 70, 75, 80, 85, 90, 92, 94, 96, 98, 99 % oder mehr Prozent aufweist, wobei bevorzugt andere als die das katalytisch aktive Zentrum ausbildende Aminosäuren oder für die Struktur oder Faltung essentielle Aminosäuren deletiert oder substituiert sind oder letztere lediglich konservativ substituiert sind, beispielsweise ein Glutamat statt einem Aspartat oder ein Leucin statt einem Valin. Es besteht keine Notwendigkeit, dass die Sequenz über ihre gesamte Länge eine entsprechend hohe Homologie aufweist, erfindungsgemäß können auch Fusionsproteine oder für solche kodierende Nukleinsäuren verwendet werden, die einen Teil mit entsprechender Homologie und/oder Aktivität aufweist. Der Stand der Technik beschreibt Algorithmen, die verwendet werden können, um das Ausmaß von Homologie von zwei Sequenzen zu berechnen, z. B. Arthur Lesk (2008), Introduction to bioinformatics, 3rd edition. In einer weiteren bevorzugteren Ausführungsform der vorliegenden Erfindung weist die Variante einer Aminosäure- oder Nukleinsäuresequenz, bevorzugt zusätzlich zur oben genannten Sequenzhomologie, im Wesentlichen die gleiche enzymatisch Aktivität des Wildtypmoleküls bzw. des ursprünglichen Moleküls auf. Zum Beispiel weist eine Variante eines als Protease enzymatisch aktiven Polypeptids die gleiche oder im Wesentlichen die gleiche proteolytische Aktivität wie das Polypeptidenzym auf, d.h. die Fähigkeit, die Hydrolyse einer Peptidbindung zu katalysieren. In einer besonderen Ausführungsform bedeutet der Begriff "im Wesentlichen die gleiche enzymatische Aktivität" eine Aktivität mit Hinblick auf die Substrate des Wildtyp-Polypeptids, die deutlich über der Hintergrundaktivität liegt oder/und sich um weniger als 3, bevorzugter 2, noch bevorzugter eine Größenordnung von den K_{M}- und/oder k_{cat}- Werten unterscheidet, die das Wildtyppolypeptid mit Hinblick auf die gleichen Substrate aufweist. In einer weiteren bevorzugten Ausführungsform umfasst der Begriff "Variante" einer Nukleinsäure- oder Aminosäuresequenz wenigstens einen aktiven Teil/oder Fragment der Nukleinsäure- bzw. Aminosäuresequenz. In einer weiteren bevorzugten Ausführungsform bedeutet der Begriff "aktiver Teil", wie hierin verwendet, eine Aminosäuresequenz oder eine Nukleinsäuresequenz, die eine geringere als die volle Länge der Aminosäuresequenz aufweist bzw. für eine geringere als die volle Länge der Aminosäuresequenz kodiert, wobei die Aminosäuresequenz oder die kodierte Aminosäuresequenz mit geringerer Länge als der Wildtyp-Aminosäuresequenz im Wesentlichen die gleiche enzymatische Aktivität wie das Wildtyppolypeptid oder eine Variante davon aufweist, beispielsweise als Alkoholdehydrogenase, Monooxygenase oder Transaminase. In einer besonderen Ausführungsform umfasst der Begriff "Variante" einer Nukleinsäure eine Nukleinsäure, deren komplementärer Strang, bevorzugt unter stringenten Bedingungen, an die Wildtyp-Nukleinsäure bindet. Die Stringenz der Hybridisierungsreaktion ist für den Fachmann leicht bestimmbar und hängt im Allgemeinen von der Länge der Sonde, den Temperaturen beim Waschen und der Salzkonzentration ab. Im Allgemeinen benötigen längere Sonden höhere Temperaturen zum Hybridisieren, wohingegen kürzere Proben mit geringen Temperaturen auskommen. Ob Hybridisierung stattfindet, hängt im Allgemeinen von der Fähigkeit der denaturierten DNA ab, an komplementäre Stränge zu annellieren, die in ihrer Umgebung vorhanden sind, und zwar unterhalb der Schmelztemperatur. Die Stringenz von Hybridisierungsreaktion und entsprechende Bedingungen sind ausführlicher in Ausubel *et al.* (1995) beschrieben. In einer bevorzugten Ausführungsform umfasst der Begriff "Variante" einer Nukleinsäure, wie hierin verwendet, eine beliebige Nukleinsäuresequenz, die für die gleiche Aminosäuresequenz wie die ursprüngliche Nukleinsäure oder eine Variante dieser Aminosäuresequenz im Rahmen der Degeneriertheit des genetischen Codes kodiert.

Für viele Anwendungen wird die Oxidoreduktase vom AlkB-Typ verwendet als Teil eines Ganzzellkatalysators die Ausführungsform der Wahl sein, da sie keine oder zumindest vollständige Aufreinigung der Oxidoreduktase bzw. ihrer Aktivität erfordert. In einer bevorzugten Ausführungsform wird unter dem Begriff "Ganzzellkatalysator", wie hierin verwendet, eine stoffwechselaktive Zelle verstanden, die eine enzymatische Aktivität von Interesse, bevorzugt die einer Oxidoreduktase vom AlkB-Typ, aufweist, bevorzugt in einem relativ zu ihrem Wildtyp erhöhten Maß, das vorteilhafterweise durch Überexpression einer rekombinanten Oxidoreduktase vom AlkB-Typ auf einem Plasmid oder integriert ins Genom erreicht werden kann. Dem Fachmann sind zahlreiche Systeme zur Herstellung von Ganzzellkatalysatoren bekannt, beispielsweise aus der DE 60216245. In einer bevorzugten Ausführungsform handelt es sich bei der als Ganzzellkatalysator oder bei der als Expressionssystem verwendeten Zelle um eine prokaryotische, bevorzugt um eine bakterielle Zelle. In einer weiteren bevorzugten Ausführungsform handelt es sich um eine Säugetierzelle. In einer weiteren bevorzugten Ausführungsform handelt es sich um eine niedere eukaryotische Zelle, bevorzugt um eine Hefezelle. Beispielhafte prokaryotische Zellen umfassen *Escherichia,* besonders *Escherichia coli,* und Stämme der Gattung *Pseudomonas* und *Corynebacterium.* Beispielhafte niedere eukaryotische Zellen umfassen die Gattungen *Saccharomyces, Candida, Pichia, Yarrowia, Schizosaccharomyces,* besonders die Stämme *Candida tropicalis, Schizosaccharomyces pombe, Pichia pastoris, Yarrowia lipolytica* und *Saccharomyces cerivisiae.* Die Zelle kann eine oder mehr als eine für ein erfindungsgemäß verwendetes Enzym kodierende Nukleinsäuresequenz auf einem Plasmid oder integriert in ihrem Genom enthalten. Verfahren zur Herstellung derartiger Plasmide bzw. Zellen kann der Fachmann routinemäßig ausführen. Sie sind in Lehrbüchern und Versuchsprotokollsammlungen der Molekularbiologie, Biochemie, Genetik und Mikrobiologie beschrieben, beispielsweise in Sambrook et al. (1989).

In einer weiteren bevorzugten Ausführungsform, handelt es sich bei der Oxidoreduktase vom AlkB-Typ jedoch um ein aufgereinigtes Enzym. Dabei kann es sich, wie bei allen erfindungsgemäß eingesetzten enzymatisch aktiven Polypeptiden, um Zellen umfassend enzymatisch aktive Polypeptide oder deren Lysate oder Präparationen der Polypeptide in sämtlichen Aufreinigungsstufen, vom kruden Lysat bis zum reinen Polypeptid, handeln. In einer bevorzugten Ausführungsform ist unter dem Begriff "aufgereinigtes" Enzym, wie hierein verwendet, in einer bevorzugten Ausführungsform zu verstehen, dass nicht die ganze Zelle oder ein unverarbeitetes Extrakt von ihr zur Katalyse eingesetzt wird, sondern das Enzym teilweise oder vollständig aufgereinigt wird. In einer besonders bevorzugten Ausführungsform bedeutet der Begriff "aufgereinigtes" Enzym, wie hierin verwendet, dass das Enzyme soweit aufgereinigt ist, dass es auf einem SDS-Gel der Präparation zunehmend bevorzugt wenigstens ca. 80, 85, 95, 98 oder bevorzugt 99 % der sichtbaren Proteine darstellt. In einer noch bevorzugteren Ausführungsform ist das Enzym soweit aufgereinigt, dass es das einzige erkennbare Polypeptid auf einem SDS-Gel der entsprechenden Präparation darstellt. Dem Fachmann auf dem Gebiet sind zahlreiche Verfahren bekannt, mit denen enzymatisch aktive Polypeptide in geeigneten Zellen überexprimiert und aufgereinigt bzw. isoliert werden können. So können zur Expression der Polypeptide sämtliche dem Fachmann zur Verfügung stehende Expressionssysteme verwendet werden, beispielsweise Vektoren vom Typ pET oder pGEX. Zur Aufreinigung kommen chromatographische Verfahren in Frage, beispielsweise die affinitätschromatographische Aufreinigung eines mit einem Tag versehenen rekombinanten Proteins unter Verwendungen eines immobilisierten Liganden, beispielsweise eines Nickelions im Falle eines Histidin-Tags, von immobilisiertem Glutathion im Falle einer an das Zielprotein fusionierten Glutathion-S-Transferase oder von immobilisierter Maltose im Falle eines Tags umfassend Maltose-bindendes Protein.

Die aufgereinigten enzymatisch aktiven Polypeptide können entweder in löslicher Form oder immobilisiert eingesetzt werden. Dem Fachmann sind geeignete Verfahren bekannt, mit denen Polypeptide an organischen oder anorganischen Festphasen kovalent oder nichtkovalent immobilisiert werden können, beispielsweise durch Sulfhydryl-Kupplungs-Chemie (z.B. Kits der Firma Pierce).

Die erfindungsgemäße Lehre kann auf eine Vielzahl von Alkanen angewendet werden. In einer bevorzugten Ausführungsform handelt es sich bei dem Begriff "Alkan", wie hierin verwendet, um einen gesättigten Kohlenwasserstoff, aus der Gruppe, die lineare und verzweigte Kohlenwasserstoffe der Summenformel CₙH₂ₙ₊₂ sowie zyklische Kohlenwasserstoffe der Summenformel CₙH₂ₙ umfasst, wobei n die Werte 1, 2, 3, 4, 5, 6, 7, 8, 9, 10, 11, 12, 13, 14, 15, 16, 17, 18, 19, 20 und mehr, bevorzugt 1 bis 5, noch bevorzugter 1 bis 4, annehmen kann. Beispielsweise umfassen Alkane mit 1 bis 4 Kohlenstoffatomen die Verbindungen Methan, Ethan, Propan, Butan und Isobutan. Die Alkane umfassen lineare Alkane, z.B. die Gruppe umfassend Methan, Ethan, Propan und Butan. In einer bevorzugten Ausführungsform handelt es sich bei dem Alkan um ein verzweigtes Alkan, bevorzugt aus der Gruppe umfassend Isobutan, 2-Methylbutan und neo-Pentan. In einer weiteren besonders bevorzugten Ausführungsform handelt es sich bei dem Alkan um ein Alkan aus der Gruppe umfassend Butan und Isobutan. In einer weiteren besonders bevorzugten Ausführungsform handelt es sich um Methylcyclobutan.

Zur Durchführung des erfindungsgemäßen Verfahrens kommen verschiedene Bedingungen in Frage. Essentiell ist die Anwesenheit von molekularem Sauerstoff als Oxidationsmittel. Der Sauerstoff kann in Form von Sauerstoffquellen wie Wasserstoffperoxid oder Kaliumpermanganat vorliegen und aus diesen *in situ* gebildet werden, besonders bevorzugt ist jedoch die Einleitung von Sauerstoffgas, noch bevorzugter in Form von Luft, in das die Oxidoreduktase umfassende flüssige Reaktionsmedium. Die Temperatur kann dabei mit der Maßgabe, dass im Falle der Verwendung einer lebenden Zelle bzw. einer Präparation geeigneter Enzyme die gewählte Zelle bzw. die gewählten Enzyme lebensfähig sind bzw. Aktivität zeigen, mehr als 20 °C, 30 °C, 40 °C, 50 °C, 60 °C, 70 °C oder mehr als 80 °C, bevorzugt bis 100 °C betragen. Dem Fachmann ist bekannt, welche Organismen bei welchen Temperaturen lebensfähig sind, beispielsweise aus Lehrbüchern wie Fuchs/Schlegel, 2007. Im Falle einer lebenden Hefezelle kann die Temperatur 5 bis 45 °C, bevorzugter 15 bis 42 °C, noch bevorzugter 20 bis 30 °C betragen. Im Falle eines Gram-negativen Bakteriums, bevorzugt eines Bakteriums aus der Familie der *Enterobacteriaceae,* am bevorzugtesten *E. coli,* kann die Temperatur 5 bis 45 °C, bevorzugter 15 bis 42 °C, noch bevorzugter 20 bis 30 °C, am bevorzugtesten 35 bis 40 °C betragen. Der pH-Wert muss derart sein, dass die Aktivität der Oxidoreduktase vom AlkB-Typ zumindest ausreichend lange erhalten bleibt. Im Falle der Verwendung eines Ganzzellkatalysators muss die Zelle ausreichend lange intakt bleiben. Beispielsweise kann der pH-Wert zwischen 3 und 12, bevorzugter 5 und 9, noch bevorzugter 6 und 8 liegen.

Das Kontaktieren des Alkans mit der Oxidoreduktase vom AlkB-Typ erfolgt bevorzugterweise in der Weise, dass die aufgereinigte oder in Form von einem Ganzzellkatalysator vorliegende Oxidoreduktase vom AlkB-Typ in wässriger Lösung in ausreichend stabiler Form vorliegt und das Alkan unter sanftem Rühren zusammen mit Sauerstoff zur Lösung hinzugegeben wird, falls es sich um ein festes oder flüssiges Alkan handelt, oder in Form eines Gases, sofern es sich um ein gasförmiges Alkan handelt, in die wässrige Lösung eingeleitet wird. Der Fachmann ist in der Lage, im Rahmen routinemäßiger Experimente stabilisierte Formen des Enzyms oder Ganzzellkatalysators bereitzustellen. Zu beachtende Faktoren wie der Einsatz eines geeigneten Puffersystems, die Einstellung geeigneter Temperatur-, pH- und Salzkonzentrationswerte sind in der Literatur beschrieben, beispielsweise in Cornish-Bowden, 1995.

Zur Anzucht der erfindungsgemäßen Zelle kommen zahlreiche Kulturmedien in Frage, im Falle der Verwendung einer Hefezelle beispielsweise YPD, YPN und YNB, die mit Aminosäuren, beispielsweise mit 0,01 g/L Tryptophan, oder mit Glucose, beispielsweise in einer Konzentration von 1 % (w/v), supplementiert sein können. Im Falle der Verwendung eines Bakteriums aus der Familie der *Enterobacteriaceae,* bevorzugt *E. coli,* kommen zur Anzucht Vollmedien wie LB-Medium oder Hochzelldichtemedium (HZD-Medium) bestehend aus NH₄SO₄ 1,76 g, K₂HPO₄ 19,08 g, KH₂PO₄ 12,5 g, Hefeextrakt 6,66 g, Na₃-Citrat 1,96 g , NH₄Fe-Citrat (1 %) 17 ml, Spurenelementelösung US3 5 ml, Feedlösung (Glucose 50 % w/v, MgSO₄ [x 7 H₂O 0,5 % w/v, NH₄Cl 2,2 % w/v) 30 ml pro Liter in Frage.

In einer bevorzugten Ausführungsform werden im erfindungsgemäßen Verfahren verwendete Zellen in einem anderen Medium als demjenigen herangezogen, das für die Alkanoxidation verwendet wird. In einer besonders bevorzugten Ausführungsform handelt es sich bei dem für die Anzucht verwendeten Medium um ein Vollmedium und um das für Alkanoxidation verwendete Medium um ein Minimalmedium. Das erfindungsgemäße Verfahren, sofern es unter Verwendung lebensfähiger Zellen durchgeführt wird, wird nach Anzucht der Zellen bevorzugt in Transformationspuffer durchgeführt, der pro Liter (NH₄)H₂PO₄ 8 g, NaCl 0,5 g, MgSO₄ x 7 H₂O 0,48 g, Spurenelementlösung US315 ml. 1 Liter der Spurenelementelösung US3 setzte sich zusammen aus HCl 37 % 36,5 g, MnCl₂ x 4H₂O 1,91 g, ZnSO₄ x 7H₂O 1,87 g, Na-EDTA x 2H₂O 0,8 g, H₃BO₃ 0,3 g, Na₂MoO₄ x 2H₂O 0,25 g, CaCl₂ x 2H₂O 4,7 g, FeSO₄ x 7 H₂O 17,8 g, CuCl₂ x 2H₂O 0,15 g umfasst und dessen pH auf 5,4 eingestellt wird. In einer weiteren Ausführungsform wird die Alkanoxidation in M9-Medium durchgeführt (15 g Glucose, 6,79 g Na₂PO₄, 3 g KH₂PO₄, 0,5 g NaCl, 2 g NH₄Cl, 15 g Hefeextrakt, 0,49 g MgSO₄*7H₂O, 1 ml TE und 50 µg Kanamycin in 1000 ml Schüttelkolben überimpft, wobei die Spurenelementlösung (TE) pro Liter wie folgt angesetzt wird: 36,5 g HCl 37 %, 1,91 g MnCl₂*4H₂O, 1,87 g ZnSO₄*7H₂O, 0,84 g Na-EDTA*2H₂O, 0,3 g H₃BO₃, 0,25 g Na₂MoO₄*2H₂O, 4,7 g CaCl₂*2H₂O, 17,3 g FeSO₄*7H₂O und 0,15 g C_{U}Cl₂*2H₂O).

Das erfindungsgemäßen Verfahrens kann bei Atmosphärendruck durchgeführt. Im Falle der Verwendung gasförmiger Alkanedukte kann es jedoch vorteilhaft sein, die Oxidoreduktase vom AlkB-Typ die Reaktion bei höheren Drücke in Anwesenheit eines Gasgemisches katalysieren zu lassen, das überwiegend eine Mischung aus Alkanen und Sauerstoff umfasst, zunehmend bevorzugt mehr als 50, 60, 70 oder 80 Volumenprozent. In einer bevorzugten Ausführungsform beträgt der Druck mehr als 1,5, 2, 3 oder 4 bar. In einer weiteren Ausführungsform beträgt der Druck 0,5 bis 4, bevorzugt 1 bis 3, am bevorzugtesten 1 bis 1,5 bar.

Ein besonderer Vorteil des erfindungsgemäßen Verfahrens besteht darin, dass bestimmte Verhältnisse der Oxidationsprodukte des als Edukt eingesetzten Alkans erhalten werden können. Das Alkan kann von der Oxidoreduktase vom AlkB-Typ an einem endständigen Kohlenstoffatom, d.h. einem Kohlenstoffatom, das kovalent nur mit höchstens einem weiteren Kohlenstoffatom direkt verbunden ist, prinzipiell zu drei Oxidationsstufen oxidiert werden, nämlich dem Alkohol, dem Aldehyd und der Carbonsäure. In einer bevorzugten Ausführungsform bedeutet die Angabe, dass "das Verhältnis von Carbonsäure zu Alkohol unter den Oxidationsprodukten bevorzugt größer als 1:1 ist", dass das Stoffmengenverhältnis von Carbonsäure zu Alkohol, bevorzugt das Stoffmengenverhältnis von durch Oxidation eines endständigen Kohlenstoffatoms entstandener Carbonsäure zu durch Oxidation eines endständigen Kohlenstoffatoms entstandenem Alkohol größer als 1:1 ist, d.h. im Produktgemisch sind mehr Moleküle der durch Oxidation eines endständigen Kohlenstoffatoms entstandenen Carbonsäure als Moleküle des durch Oxidation eines endständigen Kohlenstoffatoms entstandenen Alkohols vorhanden.

In einer bevorzugten Ausführungsform kann eine Oxidoreduktase vom AlkB-Typ, bevorzugt AlkB von *Pseudomonas putida* Gpo1, verwendet werden, um aus einem Alkan, bevorzugt einem mit 1 bis 5, noch bevorzugter 1 bis 4 Kohlenstoffatomen, am bevorzugtesten 4 Kohlenstoffatomen, in Anwesenheit von Sauerstoff Oxidationsprodukte herzustellen, wobei das Verhältnis von Carbonsäure zu Alkohol bevorzugt größer als 1:1, zunehmend bevorzugter 1,5:1, 2:1, 5:1, 10:1, 15:1 oder 20:1.

In einer weiteren bevorzugten Ausführungsform umfasst die Erfindung ein Verfahren zur Oxidation eines endständigen Kohlenstoffatoms eines nichtzyklischen Alkans zu einem entsprechenden Aldehyd und/oder einer entsprechenden endständigen Monocarbonsäure, umfassend Kontaktieren des Alkans mit einem biologischen Agens, das eine katalytisch aktive Oxidoreduktase umfasst, in Anwesenheit von einem Oxidationsmittel, wobei es sich bei dem Alkan um Butan oder Isobutan handelt und wobei es sich bei der Oxidoreduktase um eine Oxidoreduktase vom alkB-Typ, noch bevorzugter um die Monooxygenase AlkB aus P. *putida* GPO1 oder ein Homologon davon handelt und das Oxidationsmittel Sauerstoff ist und eine Verwendung einer Oxidoreduktase vom alkB-Typ, bevorzugter um die Monooxygenase AlkB aus *P. putida* GPO1 oder einem Homologon davon zur Oxidation eines endständigen Kohlenstoffatoms eines nichtzyklischen Alkans zu einem entsprechenden Aldehyd und/oder einer entsprechenden endständigen Monocarbonsäure, wobei es sich bei dem Alkan um Butan oder Isobutan handelt.

Die vorliegende Erfindung wird weiterhin durch die folgenden Figuren und nicht beschränkenden Beispiele veranschaulicht, denen weitere Merkmale, Ausführungsformen, Aspekte und Vorteile der vorliegenden Erfindung entnommen werden können.

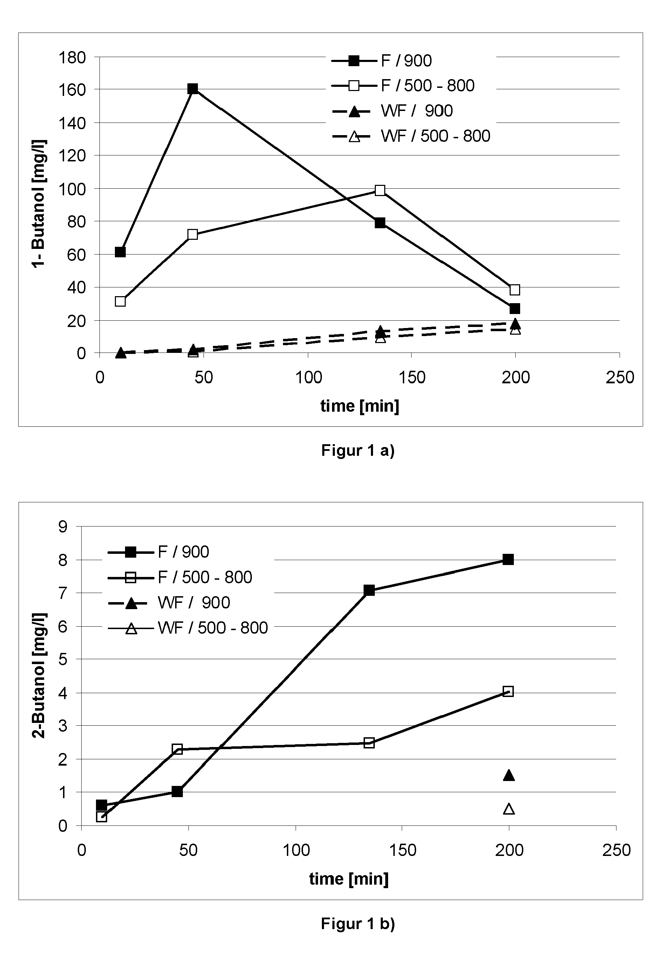

**Figs. 1a****), b), c)** und **d)** zeigen die Konzentration von 1-Butanol (**a)**), 2-Butanol (**b)**), Butyraldehyd (**c)**) bzw. Buttersäure (**d)**) im Zeitverlauf bei Umsetzung von Butan mit Sauerstoff durch das Monooxygenase-System alkBGT aus *P. putida* GPO1 bei einer Rührerdrehzahl von 500 - 800 oder 900 Upm. Es werden die Konzentrationen im Fermenter (F) und in der Waschflasche (WF) gezeigt.

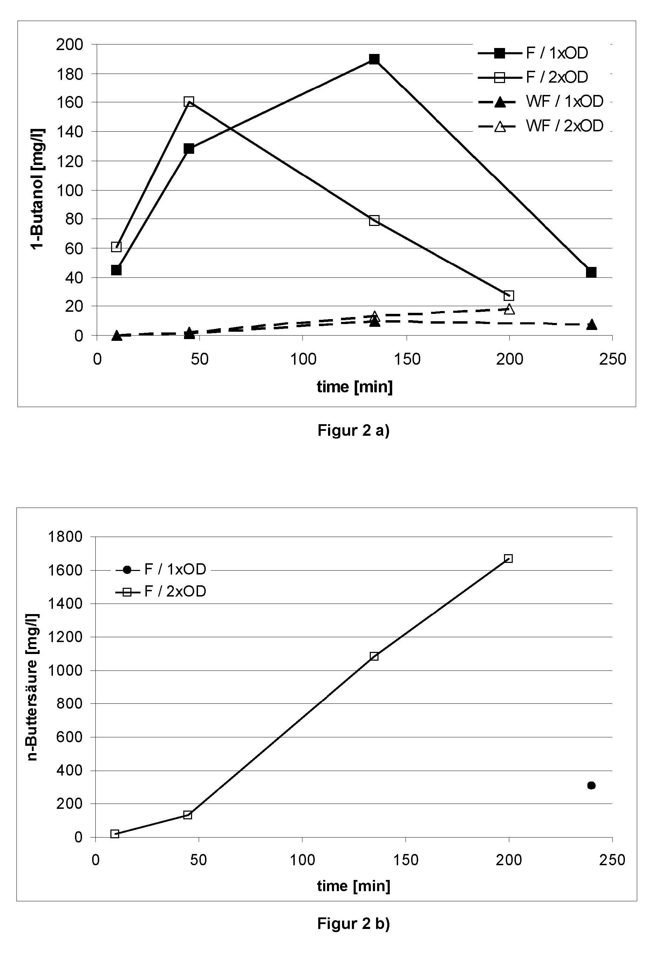

**Figs. 2** a) und **b)** zeigen den Einfluss der Biomassekonzentration auf die Oxidation von Butan durch E. *coli* durch das Monooxygenase-System alkBGT aus P. *putida* GPO1, genauer die Konzentration von 1-Butanol (**a)**) und Buttersäure (**b)**) im Zeitverlauf.

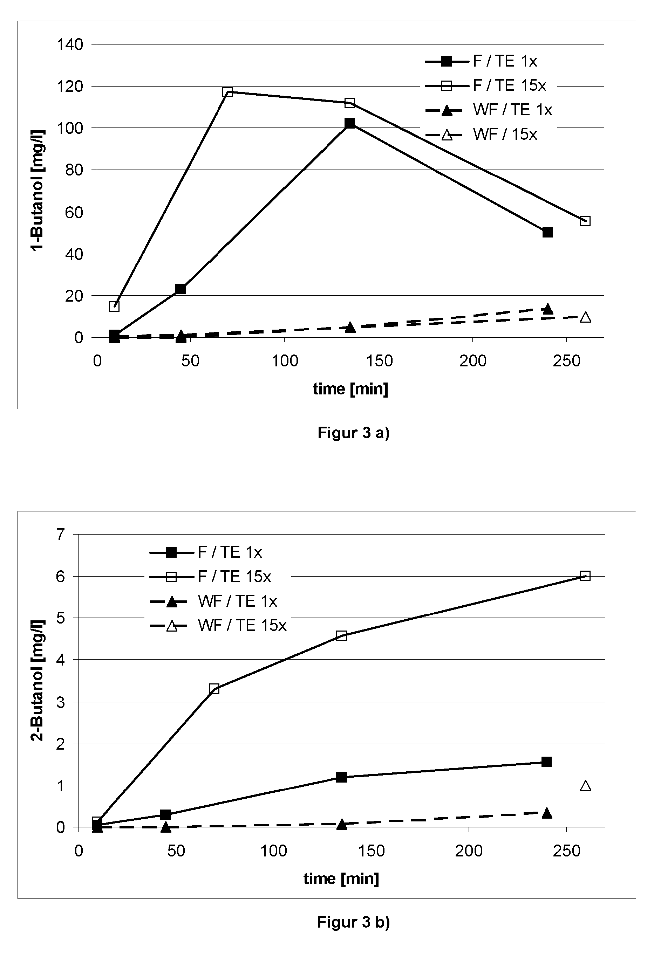

**Figs. 3a****), b), c)** und **d)** zeigen den Einfluss der Konzentration an Spurenelement (TE)-Lösung auf die Oxidation von Butan durch E. *coli* durch das Monooxygenase-System alkBGT aus P. *putida* GPO1, genauer die Konzentration von 1-Butanol (**a)**), 2-Butanol (**b)**), Butyraldehyd (**c)**) bzw. Buttersäure (**d)**) im Zeitverlauf

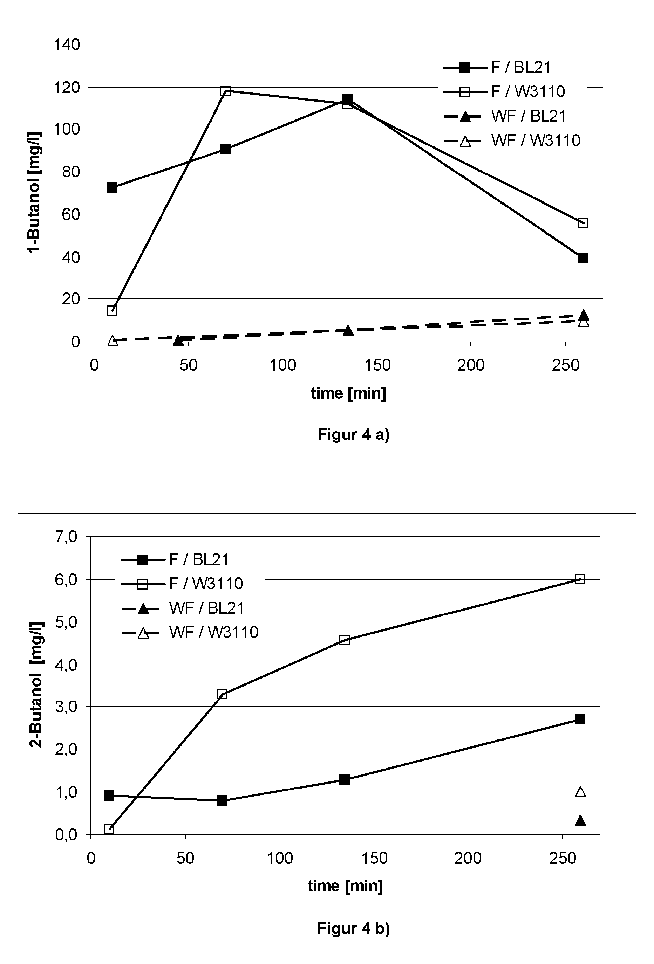

**Figs. 4a****), b), c)** und **d)** zeigen einen Stämmevergleich E. *coli* BL21 mit E. *coli* W3110 durch das Monooxygenase-System alkBGT aus *P. putida* GPO1 und deren Einfluss auf die Oxidation von Butan durch E. *coli* mit der Monooxygenase (alkBGT) aus *P. putida* GPO1, genauer die Konzentration von 1-Butanol (**a)**), 2-Butanol (**b)**), Butyraldehyd (**c)**) bzw. Buttersäure (**d)**) im Zeitverlauf

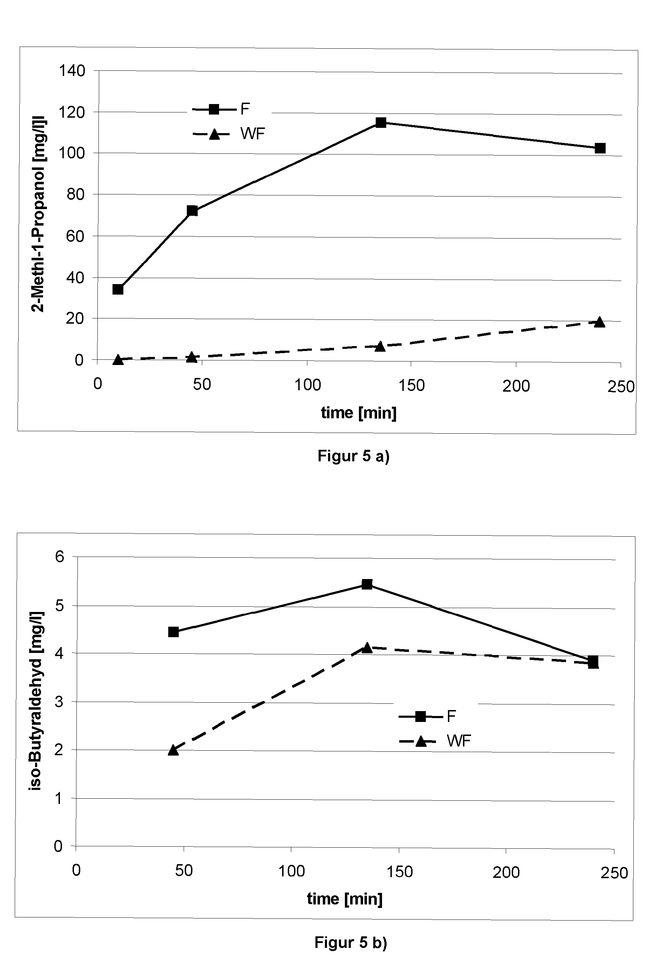

**Figs. 5a****)**, **b), c)** zeigen die Oxidation von iso-Butan durch E. *coli* durch das Monooxygenase-System alkBGT aus *P. putida* GPO1, genauer die Konzentration von 2-Methyl-1-propanol (**a)**), iso-Butyraldehyd (**b)**) und iso-Buttersäure (**c)**) im Zeitverlauf.

### Beispiel 1: Oxidation von Butan durch E. coli durch das Monooxygenase-System alkBGT aus P. putida GPO1

100µl einer Glycerin-Kryokultur von E. *coli* BL21 pCOM10 (Leerplasmid) und E. *coli* BL21 pBT10 (WO 2009/077461) werden auf einer LB-Agarplatte mit 50 µl Kanamycin ausplattiert und für 24 h bei 37°C inkubiert. Die LB-Platten werden aus 1 Liter einer Lösung von Hefeextrakt 5 g, Pepton 10 g, NaCl 0,5 g, Agar-Agar 15 g und Kanamycin 50 µg hergestellt. Der pH wird vor dem Autoklavierten mit 5 % NH₄OH auf 7,4 eingestellt.

Von diesen Platten werden (für einen Konversionsansatz) 2 x 25 ml LB-Broth (obige Lösung ohne Agar-Agar) mit 50 µl Kanamycin in einem 100 ml-Schüttelkolben mit Schikanen mit einem vollen Loop einer Impföse (Kapazität 10 µl) angeimpft. Die Kulturen werden für 24 h bei 37 °C und 200 Upm (Amplitude 2,5 cm) inkubiert.

Danach werden je 25 ml der Kulturbrühe in 75 ml modifiziertes M9-Medium (steril filtriert) mit folgender Zusammensetzung pro Liter: 15 g Glucose, 6,79 g Na₂PO₄, 3 g KH₂PO₄, 0,5 g NaCl, 2 g NH₄Cl, 15 g Hefeextrakt, 0,49 g MgSO₄*7H₂O, 1 ml TE und 50 µg Kanamycin in 1000 ml Schüttelkolben überimpft. Die Spurenelementlösung (TE) wird pro Liter wie folgt angesetzt: 36,5 g HCl 37 %, 1,91 g MnCl₂*4H₂O, 1,87 g ZnSO₄*7H₂O, 0,84 g Na-EDTA*2H₂O, 0,3 g H₃BO₃, 0,25 g Na₂MoO₄*2H₂O, 4,7 g CaCl₂*2H₂O, 17,3 g FeSO₄*7H₂O und 0,15 g CuCl₂*2H₂O. Der pH wird mit 5 % NH₄OH auf 7,4 eingestellt. Des Weiteren werden pro Kolben 3 Tropfen autokaviertem Antischaum (Delamex) und zugegeben.

Die Kolben werden für 2 Stunden bei 37°C und 180 Upm (Amplitude 2,5 cm) inkubiert. Danach wird die Temperatur auf 25 °C reduziert. Nach 0,5 Stunden bei 25 °C erfolgt die Induktion mit 0,4 mM DCPK. Die Kultur wird für weitere 16 Stunden bei 25 °C und 180 Upm geschüttelt. Danach erfolgt eine mikroskopische Prüfung auf Monosepsis.

Die Kulturen werden vereinigt, in 50 ml-Falcon Tubes abgefüllt und bei 10000 g bei 25 °C für 10 Minuten zentrifugiert. Der Überstand wird verworfen. Die Pellets aus 200 ml Kultur werden in 10 ml Konversionspuffer resuspendiert. Der Konversionspuffer besteht aus 70 mM Na⁺/K⁺ phosphatpuffer, pH 7, eingestellt mit 1 M NaOH, mit 6,79 g Na₂PO₄, 3 g KH₂PO₄, 0,5 g NaCl, 0,49 g MgSO₄*7H₂O, 1 ml TE und 50 µg Kanamycin oder aus 70 mM (NH₄)H₂PO₄ Puffer, pH 7 mit 8 g (NH₄)H₂PO₄, 0,5 g NaCl, 0,49 g MgSO₄*7H2O, 1 ml TE und 50 µg Kanamycin pro Liter. Die pH-Einstellung erfolgt hier mit 5 % NH₄OH.

170 ml Puffer mit ca. 3 Tropfen autokaviertem Antischaum (Delamex) werden in einem 300 ml-Fermenter vorgelegt. Der Fermenter wird mit einem Gasgemisch aus einer Gasflasche mit 5 bar Anfangsdruck von 25 % Butan und 75 % synthetischer Luft über einen Sinterglasperlator mit einer Porengröße von 0,2 µm mit einer Fußrate von 25 l/h begast. Der Fermenter wird in einem Wasserbad auf 25 °C temperiert und mit Hilfe eines Magnetrührers mit 500 Upm für 2 Stunden, danach mit 800 Upm gerührt. Die Abluft wird durch eine Waschflasche, die mit 150 ml Wasser gefüllt ist, abgeleitet.

Der Fermenter wird mit 10 ml des resuspendierten Pellets angeimpft. Die OD beider Kulturen liegt bei ca. 10. Die Reaktion wird durch Zugabe von 1 Vol.-% Glucose gestartet. Der pH kann wahlweise über den Versuchszeitraum ungeregelt oder geregelt sein. Nach 10, 45, 135 und 240 Minuten werden aus dem Fermenter und der Waschflasche jeweils 10 ml Probe gezogen. In den Proben aus dem Fermenter wird mit 2 ml HCl die Reaktion gestoppt. Die Fermenterproben werden 10 Minuten bei 10000 g und Raumtemperatur zentrifugiert und der Überstand über einen 0,2 µm-Spritzenvorsatzfilter filtriert. Die Proben werden für die Analytik in HPLC Vials abgefüllt. Die Analytik erfolgte chromatographisch mit HPLC-RID an einer Agilent Technologies 1200-Anlage. Es wurde eine Aminex HPX-87H-Säule (300mmx 7,8mm) verwendet. Die Anlage wurde mit 10 mM H₂SO₄ als Laufmittel bei einer Flussrate von 0,6 ml/min und einer Säulentemperatur von 40°C betrieben. Standards aller zu analysierender Stoffe wurden in Reinstwasser vorbereitet und unter identischen Bedingungen gemessen. Die Auswertung erfolgte über Vergleich der Retentionszeiten. Zusätzlich wird zu jedem Probenahmezeitpunkt eine 2 ml-Probe zu Bestimmung von pH, OD und Glucosekonzentration aus dem Fermenter gezogen. Der pH wird an einem externen pH-Meter gemessen, die OD spektrometrisch bei 600 nm und der Glucosegehalt mit einem biochemischen Analysator (YSI Select 2700 Von Kreienbaum) bestimmt.

### Ergebnisse

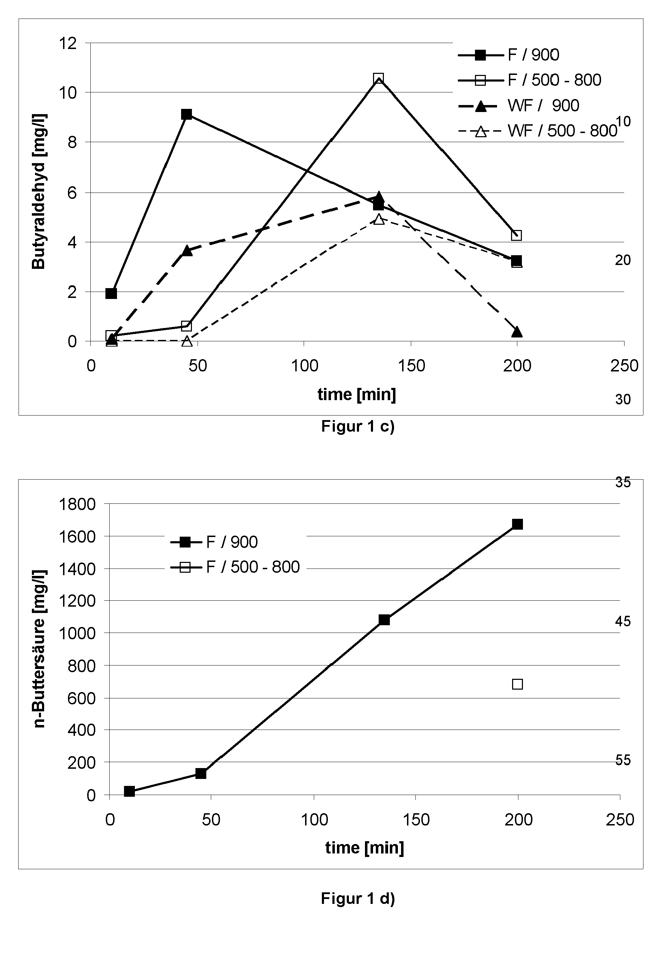

Die Ergebnisse sind in den **Fig. 1 a)** **- d)** dargestellt. Bei den Versuchen mit E. *coli* BL21 pCOM10 (Leerplasmid) findet keine Oxidation von Butan bzw. 1-Butanol statt. Hingegen werden mehr Verwendungen von E. *coli* BL21 pBT10 als Oxidationsprodukten von n-Butan: 1-Butanol, Buttersäure, 2-Butanol, Butyraldehyd, 1,4-Butandiol (nicht quantifizierbar) und Butyrolacton (in Spuren) gefunden.

Die Konzentration aller Oxidationsprodukte steigt über den gesamten Versuchszeitraum an. Es wird ca. 1 g/lh Glucose verbraucht, der pH-Wert sinkt von 7 auf ca 5.

### Beispiel 2: Einfluss der Rührerdrehzahl (Stofftransportlimitierung) auf die Oxidation von n-Butan durch E. coli mit der Monooxygenase (alkBGT) aus P. putida GPO1

Der Versuch wird analog dem Beispiel 1 durchgeführt. Die Rührerdrehzahl wird in einem 2. Ansatz von Beginn an konstant auf 900 Upm gestellt. Die OD ist im Vergleich zu Beispiel 1 doppelt so hoch. Die TE-Konzentration ist 15fach. Die letzte Probenahme erfolgte nach 200 Minuten.

### Ergebnisse

Die Bildung von 1-Butanol im Fermenter (F) erfolgt bei ständig höherer Drehzahl schneller und erreicht eher ihr Maximum. In den Waschflaschen (WF) ist die Konzentration von 1-Butanol auf niedrigem Niveau annähernd identisch. Die Konzentration von 2-Butanol im Fermenter (F) steigt bei jeder Rührerdrehzahl über den gesamten Versuchszeitraum an, bleibt aber gering. In den Waschflaschen ist 2-Butanol erst am Ende der Versuchszeit nachweisbar. Die Konzentration von Butyraldehyd steigt mit höherer Rührerdrehzahl schneller, wird aber da der Dampfdruck bei 113 hPa (20°C) liegt auch schneller ausgetrieben. Butyraldehyd ist so nur qualitativ, nicht aber quantitativ nachweisbar.

n-Buttersäure wird bei niederer Rührerdrehzahl erst am Ende des Versuchszeitraums gebildet. Mit hoher Rührerdrehzahl steigt die Konzentration kontinuierlich an. In den Waschflaschen kann keine n-Buttersäure nachgewiesen werden.

### Beispiel 3: Einfluss der Biomassekonzentration auf die Oxidation von n-Butan durch E. coli mit der Monooxygenase (alkBGT) aus P. putida GPO1

Der Versuch wird analog dem Beispiel 1 durchgeführt. Die Rührerdrehzahl liegt konstant bei 900 Upm, die TE Konzentration ist jeweils 15fach. 1x bedeutet eine OD von ca. 10, 2x entsprechend 20.

### Ergebnisse

Die Ergebnisse sind in den **Fig. 2 a)** und **b)** dargestellt. Die Maximalkonzentration wird mit doppelter OD zu einem früheren Versuchzeitpunkterreicht erreicht. 1-Butanol wird dann auch schneller umgesetzt.

Buttersäure kann nur im Fermenter (F), nicht in den Waschflaschen nachgewiesen werden. Mit der doppelten OD beginnt die Buttersäurebildung bereits zu Beginn der Konversion. Mit einfacher OD kann unter diesen Bedingungen erst nach 240 Minuten Buttersäure nachgewiesen werden. Die Konzentration beträgt etwa 18% der Maximalkonzentration bei doppelter OD.

### Beispiel 4: Einfluss der TE-Konzentration auf die Oxidation von n-Butan durch E. coli mit der Monooxygenase (alkBGT) aus P. putida GPO1

Der Versuch wird analog dem Beispiel 1 durchgeführt. Die Rührerdrehzahl beträgt konstant 900 Upm. Der verwendete Stamm ist *E*. *coli* W3110 pBT10. Die Konzentration an TE ist 1 ml/l Puffer (1x) bzw. 15 ml/l Puffer (15x). Im Versuch mit der 15fachen Konzentration sind zusätzlich mit 30 mg/l MOPS zugegeben.

### Ergebnisse

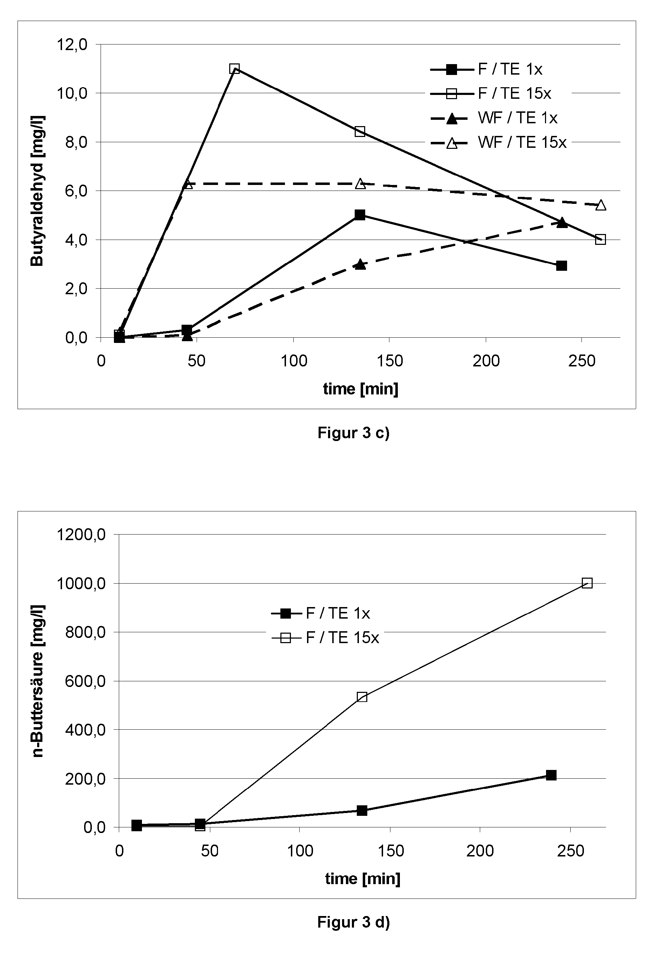

Die Ergebnisse werden sind in den **Figs. 3 a)** **- d)** dargestellt. Bei der 15 fachen TE Konzentrationen werden alle Oxidationsprodukte schneller und in höherer Konzentration gebildet.

### Beispiel 5: Stämmevergleich E. coli BL21 mit E. coli W3110 mit der Monooxygenase (alkBGT) aus P. putida GPO1

Der Versuch wird analog dem Beispiel 1 mit fester Rührerdrehzahl 900 Upm durchgeführt. Die TE-Konzentration beträgt 15 ml/l Konversionspuffer.

### Ergebnisse

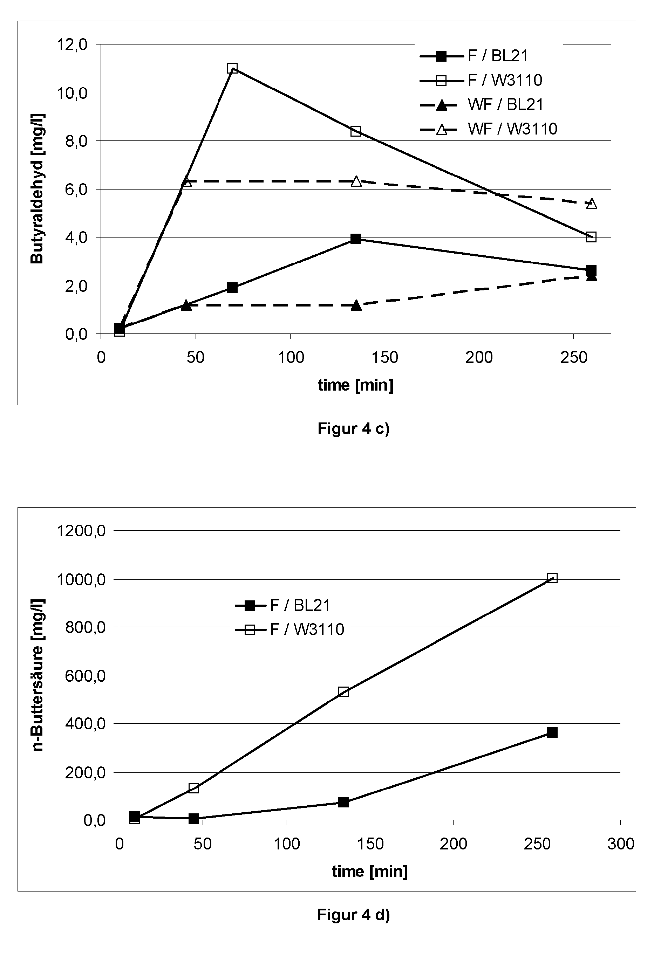

Die Ergebnisse sind in den **Figs. 4 a)** **- d)** dargestellt. Der Stamm *E. coli* W3110 pBT10 bildet alle Oxidationsprodukte schneller und in höheren Konzentrationen als der Stamm E. *coli* BL21 pBT10.

### Beispiel 6: Oxidation von iso-Butan durch E. coli mit der Monooxygenase (alkBGT) aus P. putida GPO1

Der Arbeitsablauf erfolgt analog dem Beispiel 1. Verwendet wird nur der Stamm *E. coli* W3110 pBT10. Der Konversionspuffer besteht aus 70 mM Na⁺/K⁺-phosphatpuffer, pH7, eingestellt mit 5 % NH₄OH, mit 6,79 g Na₂PO₄, 3 g KH₂PO₄, 0,5 g NaCl, 0,49 g MgSO₄*7H₂O, 15 ml TE und 50 µg Kanamycin pro Liter.

Die Begasung erfolgt wie in Beispiel 1 nur mit einer Mischung aus 25 % i-Butan und 75 % synthetischer Luft.

### Ergebnisse

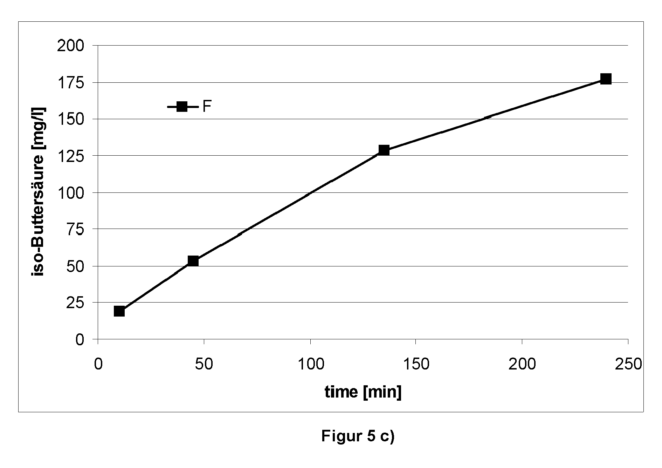

Die Ergebnisse sind in den Figuren **5 a)** **- c)** dargestellt. An Oxidationsprodukten von i-Butan werden i-Butanol, i-Buttersäure, tert.-Butanol und i-Butyraldehyd gefunden.

### Literaturstellen:

A. Cornish-Bowden (1995), Fundamentals of Enzym Kinetics, Portland Press Limited, 1995
DE 60216245 (2007): Functional Display of Polypeptides
Sambrook/Fritsch/Maniatis (1989): Molecular Cloning: A Laboratory Manual, Cold Spring Harbor Laboratory Press, 2nd edition
Fuchs/Schlegel (2007) Allgemeine Mikrobiologie, 2008, Georg Thieme Verlag
EP 98137 (1984) A microbiological process for the oxidation of alkanes, vinyl compounds and secondary alcohols
WO 2009/077461: ω-Aminocarbonsäuren oder ihre Lactame, herstellende, rekombinante Zellen
A. Lesk (2008), Introduction to bioinformatics, 3rd edition
F. M. Ausubel (1995), Current Protocols in Molecular Biology. John Wiley & Sons, Inc.
C Grant, J M Woodley, F Baganz (2011): Enzyme and Microbial Technology 48, 408-486

## Patentansprüche

1. Verfahren zur Oxidation eines Alkans umfassend Kontaktieren des Alkans mit einer Oxidoreduktase vom AlkB-Typ in Anwesenheit von Sauerstoff.

2. Verfahren nach Anspruch 1, wobei es sich bei dem Alkan um ein Alkan mit 1 bis 5 Kohlenstoffatomen handelt.

3. Verfahren nach Anspruch 2, wobei es sich bei dem Alkan um ein Alkan mit 1 bis 4 Kohlenstoffatomen, bevorzugt um Butan, handelt.

4. Verfahren nach einem der Ansprüche 1 bis 3, wobei es sich bei dem Alkan um ein verzweigtes Alkan, bevorzugt mit vier oder fünf Kohlenstoffatomen, noch bevorzugter Isobutan, handelt.

5. Verfahren nach einem der Ansprüche 1 bis 4, wobei es sich bei der Oxidoreduktase vom AlkB-Typ um AlkB aus *Pseudomonas putida* GPO1 oder eine Variante davon handelt.

6. Verfahren nach einem der Ansprüche 1 bis 5, wobei die Oxidoreduktase vom AlkB-Typ in Form von einem Ganzzellkatalysator bereitgestellt wird.

7. Verfahren nach einem der Ansprüche 1 bis 5, wobei die Oxidoreduktase vom AlkB-Typ in Form von einem aufgereinigtem Polypeptid bereitgestellt wird.

8. Verwendung einer Oxidoreduktase vom AlkB-Typ zur Herstellung in Anwesenheit von Sauerstoff eines Gemisches von Oxidationsprodukten eines Alkans, wobei das Verhältnis von Carbonsäure zu Alkohol unter den Oxidationsprodukten größer als 1:1 ist.

9. Verwendung nach Anspruch 8, wobei es sich bei dem Alkan um ein Alkan mit 1 bis 5 Kohlenstoffatomen handelt.

10. Verwendung nach Anspruch 9, wobei es sich bei dem Alkan um ein Alkan mit 1 bis 4 Kohlenstoffatomen, bevorzugt um Butan, handelt.

11. Verwendung nach einem der Ansprüche 8 bis 10, wobei es sich bei dem Alkan um ein verzweigtes Alkan, bevorzugt mit vier oder fünf Kohlenstoffatomen, noch bevorzugter Isobutan, handelt.

12. Verwendung nach einem der Ansprüche 8 bis 11, wobei es sich bei der Oxidoreduktase vom AlkB-Typ um AlkB aus *Pseudomonas putida* GPO1 oder eine Variante davon handelt.

13. Verwendung nach einem der Ansprüche 8 bis 12, wobei die Oxidoreduktase vom AlkB-Typ in Form von einem Ganzzellkatalysator bereitgestellt wird.

14. Verwendung nach einem der Ansprüche 8 bis 12, wobei die Oxidoreduktase vom AlkB-Typ in Form von einem aufgereinigten Polypeptid bereitgestellt wird.

15. Verwendung nach einem der Ansprüche 8 bis 14, wobei das Verhältnis von Carbonsäure zu Alkohol unter den Oxidationsprodukten größer als 5:1, bevorzugt größer als 12:1 ist, noch bevorzugter größer als 20:1, am bevorzugtesten größer als 40:1 ist.
